# EUROPEAN PATENT APPLICATION

(11) **EP 4 258 030 A1**
(43) Date of publication of application: **11.10.2023**
(21) Application number: 22166842.9
(22) Date of filing: 05.04.2022
(51) Int. Cl.: G02B 6/38, C12M 1/34, C12M 1/32, G02B 6/36

(54) **OPTICAL FIBRE TERMINATOR**

(71) Applicant: The Automation Partnership (Cambridge) Ltd., Hertfordshire SG8 5WY (GB)
(72) Inventor: SULLIVAN, Sean, Baldock, SG7 6TW (GB)
(74) Representative: Mewburn Ellis LLP

(57) **Abstract**

The present invention provides an optical fibre terminator for terminating two parallel optical fibres, each optical fibre comprising an optical waveguide core surrounded by a protective polymer jacket. The terminator includes a housing having a front face and a backshell. The housing is configured for insertion thereinto of the two parallel optical fibres so that respective end faces of the two optical fibres are located at predetermined spaced positions relative to the front face and the two optical fibres extend rearwardly away from the front face through the backshell. The terminator also includes a gripping spacer insertable into a matching channel formed in the backshell behind the front face and between the predetermined spaced positions such that, on insertion, the gripping spacer grips jacketed flanks of the two optical fibres on opposite sides of the spacer to thereby space the fibres apart and retain the fibres in the housing with the fibre end faces locked at the predetermined spaced positions.

## Description

### Field of the Invention

The present invention relates to an optical fibre terminator.

### Background

Cell culture is a process for growing cells in an artificial environment such as a bioreaction vessel. Typically, the cells are grown whilst suspended in a culture growth medium. Monitoring the environment to which the cells are exposed in the bioreaction vessel is important for controlling this environment, and ultimately the physiology of the cells and the amount of target produced. In particular, monitoring of parameters such as DO (dissolved oxygen) and pH of the culture growth medium within the bioreaction vessel is key to exerting this control.

Monitoring of such parameters can be achieved invasively and/or non-invasively. Invasive monitoring generally uses a sensor probe inserted directly into the culture growth medium contained in the vessel. However, this can create sensor calibration difficulties and can increase a risk of cross-contamination between vessels. In contrast, non-invasive monitoring sensors are not placed in direct contact with the culture growth medium, but may, for example, be positioned outside the vessel. While this avoids some of the problems associated with invasive monitoring, it is critical that non-invasive sensors located outside the vessel are arranged correctly in relation to the vessel to properly calibrate the sensor and also to provide accurate and consistent measurements in line with previous calibrations.

Further challenges associated with monitoring arise in relation to parallel cell culturing using large numbers of relatively small bioreaction vessels. Such an approach to cell culturing can be used to mimic larger scale cell culture environments and can improve the ratio of success and speed to market of new biopharmaceutical products by enabling screening of large numbers of potential cell clones in small-scale production-like processes. The most suitable clones can then be identified and taken forward to larger-scale development processes. Bioreaction vessels for use in such screening trials are not only small in scale, but typically are tightly arranged together and may be consumable items. However, monitoring parameters of the environment within each small-scale bioreaction vessel imposes constraints on sensor technologies. In particular, when using non-invasive probes, it is necessary to satisfy both stringent probe size limitations while also precisely and securely arranging large numbers of the probes relative to the bioreaction vessels in confined spaces.

Optical fibres comprising an optical waveguide core surrounded by a protective jacket can be used as non-invasive sensor elements to monitor parameters of the cell cultures contained in such multi-vessel bioreactor systems. However, the jackets are usually made of soft plastic, typically polyurethane or polyethylene, which is not bonded to the optical fibre. This can cause difficulties with locating and robustly securing optical fibres in correct alignment with a vessel for parameter monitoring, as particularly towards the ends of the fibres the jacket can slide relative to the optical waveguide core. To complicate matters further, soft plastics such as polyurethane or polyethylene generally cannot be glued, while piercing the jacket to secure directly to the core can create stress points in the optical waveguide core and cause it to shear.

A known approach to securing optical fibres in a desired position involves threading the fibres through a clamp plate positioned underneath a bioreaction vessel and, when the fibres are located at the desired position for parameter monitoring, tightening retaining screws on the clamp plate. The fibres are thus secured, and risk of damage thereto is mitigated by respective O-rings positioned at each fibre location on the plate, the O-rings deforming under screw tightening to mediate the clamping force between the plate and the fibre. However, such a clamp plate configuration is not generally suitable for small-scale and closely spaced bioreaction vessels. In particular, the O-rings impose a lower limit on the possible spacing between fibres on the clamp plate, which in turn imposes lower limits on the size and spacing of the vessels. Furthermore, the optical fibres can be difficult to replace without access to the retaining screws, making fibre replacement a complicated and time-consuming procedure.

An alternative approach involves removing a portion of the protective jacket of the optical fibre to expose a portion of the optical waveguide core that can then be glued to a suitable mounting material to secure the optical fibre in a desired position. However, this typically requires special glues, materials and tooling, and careful handling procedures as waveguide cores are highly brittle, and further complicates the replacement of damaged or malfunctioning fibres. For these reasons, such an approach is not generally adopted.

Thus, securing optical fibres in a desired position for performing parameter monitoring of cell cultures contained in a small-scale bioreaction vessels presents a significant technical challenge.

### Summary of the Invention

In general terms, the present invention provides a coupler for coupling two parallel elongate members, each elongate member, the coupler including:
a housing having a front face and a backshell, and being configured for insertion thereinto of the two parallel elongate members so that respective end faces of the two elongate members are located at predetermined spaced positions relative to the front face and the two elongate members extend rearwardly away from the front face through the backshell; and
a gripping spacer insertable into a matching channel formed in the backshell behind the front face and between the predetermined spaced positions such that, on insertion, the gripping spacer grips jacketed flanks of the two elongate members on opposite sides of the spacer to thereby space the elongate members apart and retain the elongate members in the housing with their end faces locked at the predetermined spaced positions.

The elongate members can be wires, tubes or pipes, for example, but more particularly the elongate members can be optical fibres, and the coupler can be a terminator for terminating the optical fibres.

Thus, in a first aspect, the present invention provides an optical fibre terminator for terminating two parallel optical fibres, each optical fibre comprising an optical waveguide core surrounded by a protective polymer jacket, the terminator including:
a housing having a front face and a backshell, and being configured for insertion thereinto of the two parallel optical fibres so that respective end faces of the two optical fibres are located at predetermined spaced positions relative to the front face and the two optical fibres extend rearwardly away from the front face through the backshell; and
a gripping spacer insertable into a matching channel formed in the backshell behind the front face and between the predetermined spaced positions such that, on insertion, the gripping spacer grips jacketed flanks of the two optical fibres on opposite sides of the spacer to thereby space the fibres apart and retain the fibres in the housing with the fibre end faces locked at the predetermined spaced positions.

Advantageously, the terminator can be easy to assemble to the fibres, and requires no gluing or bonding to connect the optical fibres, thereby facilitating replacement of individual damaged or malfunctioning fibres. Moreover, the terminator does not require the polymer jackets to be pierced in order to grip the fibres and therefore reduces the risks of structural damage to the optical waveguide cores. Rather, the terminator can firmly and safely retain the fibres via the gripping spacer e.g. to prevent any unintentional withdrawal of the fibres from the housing. Thus, the terminator reduces costs and increases reliability compared to conventional fibre attachment solutions discussed above. Additionally, the present terminator can be made compact and is thus useable even with small-scale and closely spaced bioreaction vessels.

The gripping spacer may have serrated opposite sides to grip the jacketed flanks of the inserted fibres. Advantageously, the serrated opposite sides can increase the resistance to fibre withdrawal provided by the gripping spacer, e.g. by biting onto the jacketed flanks of the fibres, thereby improving the fibre retention within the housing.

Another option is for the gripping spacer to be made of a rubber or elastomeric material that is pulled into the matching channel, the pulling stretching the material and causing the gripping spacer to contract transversely so that, when released to its unstretched state, the gripping spacer expands transversely to grip the jacket and retain it by frictional interaction therewith.

The gripping spacer may be removably insertable. Advantageously, this can facilitate the replacement of damaged or malfunctioning fibres. For example, to replace a fibre, the spacer may be removed such that it no longer retains the fibres in the housing, and a selected fibre can then easily be removed by extracting it from the terminator. Similarly, a replacement fibre can then be inserted in the same position and the fibres re-secured by re-inserting the gripping spacer between the fibres.

The gripping spacer may have an obround cross section perpendicular to its insertion direction, the sides of the spacer which grip the jacketed flanks of the two optical fibres being the straight edges of the obround cross section. Advantageously, this shape can provide a relatively large contact area between the gripping spacer and the jacketed fibres to spread loads over the jacketed flanks and reduce risk of damage to the fibres.

The front face of the housing may include respective openings to receive the fibre end faces and to define the predetermined spaced positions. For example, the optical fibre terminator may be configured such that when the fibre end faces are located at the predetermined spaced positions, the fibre end faces are flush with the front face of the housing. Advantageously, this can ensure a close contact between the fibre end faces and a wall of a bioreaction vessel, which can in turn reduce a risk of compromised signal phase and amplitude and/or loss of control during measurements when using the fibres as non-invasive probes.

The optical fibre terminator may be a duplex fibre terminator for terminating just two parallel optical fibres. For example, a duplex fibre terminator may connect to a first optical fibre for monitoring pH levels of a cell culture and to a second optical fibre for monitoring DO levels of a cell culture.

The optical fibre terminator may further include a sealing element configured to seal the terminator to a docking port. Conveniently, the sealing element may be located in a matching groove formed in the backshell of the terminator. For example, the sealing element may be an O-ring.

In a second aspect, the present invention provides a pair of terminated optical fibres terminated with the terminator according to the first aspect.

The polymer jackets of the optical fibres may be formed of polyethylene or polyurethane. Polyethylene and polyurethane are both soft plastics suitable for protecting the optical fibres from structural damage while allowing them to bend and flex as required.

Each of the terminated optical fibres may have a fibre diameter of 3 mm or less, and preferably of 2 mm or less. Advantageously, such a fibre diameter can ensure compatibility with small-scale bioreaction vessels.

A ratio of the centre-to-centre spacing between the end faces of the fibres at the predetermined spaced positions to the diameter of the fibres may be 2 or less, and preferably 1.7 or less. Again, such a ratio can also ensure compatibility with small-scale bioreaction vessels.

The terminated optical fibres may further have a retention sleeve located rearwards of and spaced from the terminator and wrapping around the optical fibres. The sleeve can help to maintain the relative positions of the fibre set by the terminator for a distance behind of the terminator and thus help to prevent the fibres being accidently pulled apart in such a way that would damage them or the terminator, or cause the fibres end to shift relative to the end face.

In a third aspect, the present invention provides a combination of a screening system and plural pairs of the terminated optical fibres according to the second aspect, wherein:
the screening system includes a plurality of sample wells for accommodating respective bioreaction vessels; and
each well includes a docking port and has the terminator of a respective pair of the terminated optical fibres docked therein such that the optical fibres of the pair can interrogate contents of a bioreaction vessel accommodated in the well via their fibre end faces.

Advantageously, such a screening system can enable screening of large numbers of potential cell clones in parallel cell-culturing processes to identify the most suitable clones for larger-scale development processes.

For example, the docking ports can be formed in the bases of the wells such that the end faces of the optical fibres are directed upwardly into the bioreaction vessels.

When the terminator of the first aspect includes a sealing element, each terminator may be sealed to its respective docking port via the respective sealing element. For example, the docking ports may have tapered profiles that progressively engage the sealing elements when the terminators are inserted therein. Advantageously, the sealing element can seal the optical fibres and parts of the screening system from spills from the bioreaction vessels, condensation and cleaning liquids.

The screening system may further include a temperature-controlled base on which the sample wells are supported, the optical fibres threading through apertures formed in the temperature-controlled base as they extend rearwardly away from the front faces of their terminators. For example, the temperature-controlled base may be formed of aluminium due to its high thermal conductivity.

In a fourth aspect, the present invention provides a method of assembling the combination according to the third aspect, the method including the steps of:
providing plural pairs of the terminated optical fibres; and
threading trailing ends of each pair of the terminated optical fibres through the docking port of the respective sample well before inserting the respective terminator into the docking port such that the optical fibres can interrogate contents of a bioreaction vessel accommodated in the well via their fibre end faces.

Advantageously, this convenient assembly method can be performed relatively quickly while also ensuring that the fibre end faces are correctly positioned to reliably interrogate contents of the bioreaction vessels. In addition, the trailing ends of the fibres can be suitably positioned for coupling to a sensing unit, e.g. a reader card for sending light signals into and reading light signals from the optical fibres.

When the combination of the third aspect is configured such that the screening system includes a temperature-controlled base, the method may further include a step of threading the trailing ends of the terminated optical fibres through the apertures formed in the base after the trailing ends have been threaded through the docking port.

The present invention includes combination of any of the aspects and optional features described, except where such a combination is clearly impermissible or expressly avoided.

### Summary of the Figures

Embodiments of the invention will now be described by way of example with reference to the accompanying drawings in which:
Figure 1 shows a perspective view of components of an optical fibre terminator according to an aspect of the present invention;
Figure 2 shows a perspective side view of the optical fibre terminator of Figure 1;
Figures 3A-3D show respectively a front elevation view, a side elevation view, a perspective view, and a plan view of a pair of terminated optical fibres terminated with the optical fibre terminator of Figure 2;
Figure 4 shows a partial plan view of a screening system;
Figure 5 shows a plan view of a multi-well screening station of the screening system of Figure 4;
Figure 6 shows a sectional view of a well and a pair of terminated optical fibres of the screening system of Figure 4; and
Figure 7 shows a perspective sectional view of part of the screening system of Figures 4 to 6.

### Detailed Description of the Invention

Aspects and embodiments of the present invention will now be discussed with reference to the accompanying figures. Further aspects and embodiments will be apparent to those skilled in the art.

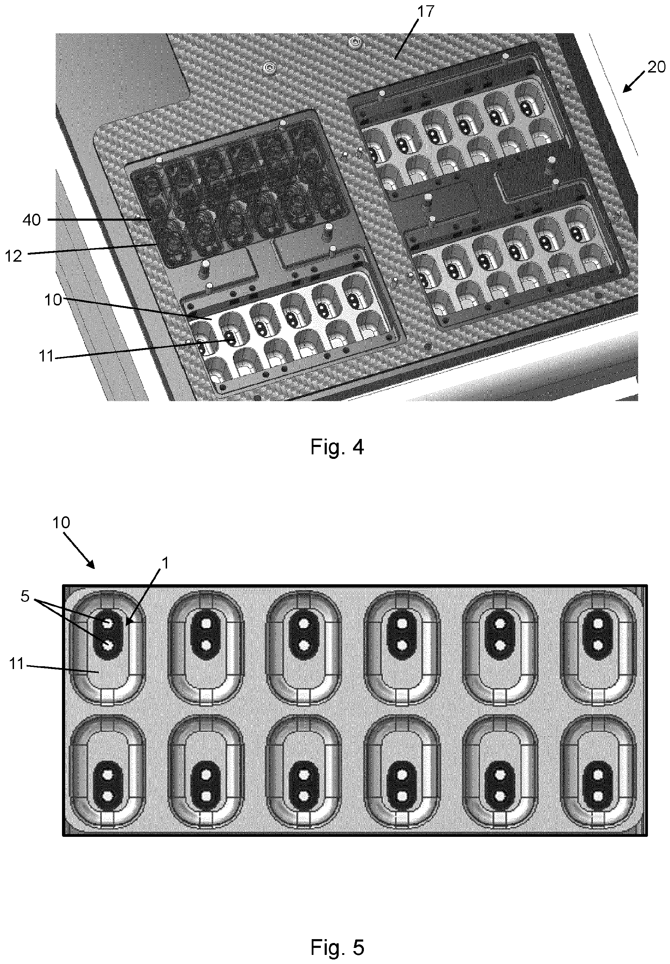

The present invention provides an optical fibre terminator for terminating optical fibres for non-invasive parameter monitoring of bioreaction contents contained in small-scale and closely spaced bioreaction vessels. The bioreaction vessels can be located in respective wells of a screening system discussed below in relation to Figures 4 to 6. In the following example the terminator is a duplex terminator for terminating just two parallel optical fibres. In particular, a first of the optical fibres can monitor pH levels of the bioreaction contents and a second of the optical fibres can monitor DO levels. The optical fibres have an optical waveguide core surrounded by a protective polymer jacket. Preferably, the fibres have a fibre diameter of 3 mm or less, and more preferably of 2 mm or less. Advantageously, such a fibre diameter ensures compatibility with small-scale and closely spaced bioreaction vessels.

The optical fibres can be commercially available fibres such as the Super Eska^{™} 2.0 mm Core Optical Fiber, 3 mm OD Polyethylene Jacket, V-2Y 1 P1960/2000 manufactured by Mitsubishi Chemical Co. These fibres have polymer jackets formed of polyethylene and their outer diameter is 3 mm.

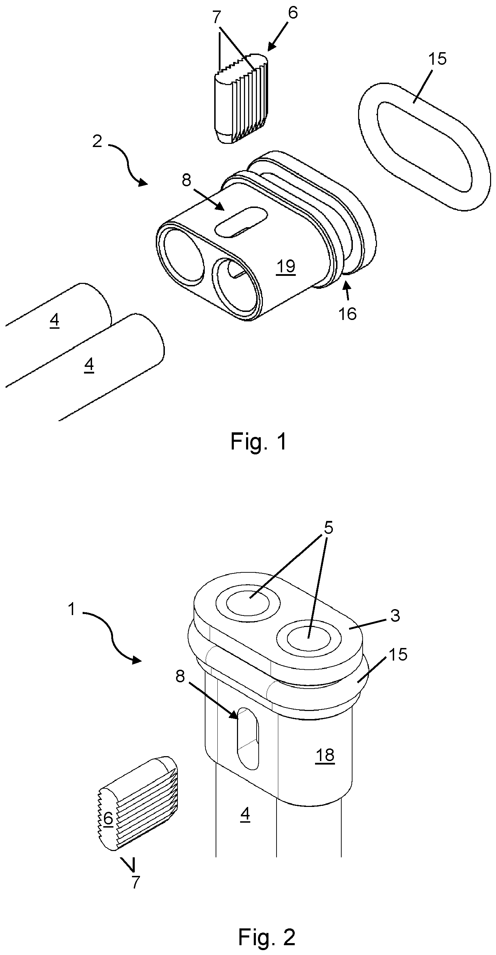

Figures 1 and 2 show perspective views respectively of (i) components of an optical fibre terminator 1 and (ii) the optical fibre terminator 1 as partially assembled. The terminator includes a housing 2 having a front face 3 (see Figure 2) and a backshell 19 behind the front face. The front face includes respective openings to receive end faces 5 of the optical fibres 4. The terminator also includes a gripping spacer 6, the backshell including a channel 8 matching the shape of the gripping spacer such that the gripping spacer is removably insertable into the channel.

The housing in this example also includes a groove 16 formed behind the front face. The groove can accommodate a sealing element, such as an O-ring 15, for sealing the terminator to a docking port of the screening system discussed below in relation to Figures 4 to 6. The housing is configured for insertion thereinto of the two parallel optical fibres 4 so that the fibre end faces 5 are located at predetermined spaced positions relative to the front face 3 of the housing and the two fibres extend rearwardly away from the front face through the backshell of the housing. As shown in Figure 2, when the fibre end faces are located at the predetermined spaced positions, they are flush with the front face. Additionally, a ratio of the centre-to-centre spacing between the end faces of the fibres at the predetermined spaced positions to the diameter of the fibres is 2 or less, and preferably 1.7 or less. In this example, the centre-to-centre spacing between the end faces of the fibres is 4 mm and the ratio is 1.3. This ensures compatibility with small-scale and closely spaced bioreaction vessels.

Next, the gripping spacer 6 is inserted into the matching channel 8 such that it grips jacketed flanks of the two optical fibres 4 on opposite sides of the spacer to thereby space the fibres apart and retain the fibres in the housing 2 with the fibre end faces 5 locked flush with the housing end face 3. Advantageously, this reduces a risk of any unintentional withdrawal of the fibres from the housing and/or displacement of the fibre end faces from their predetermined spaced positions. The fibre retention is further enhanced by an obround cross-section of the spacer perpendicular to its insertion direction, the sides 7 of the spacer which grip the jacketed flanks of the two optical fibres 4 being the straight edges of the obround cross section. This configuration provides a relatively large contact area between the sides of the spacer and the jacketed flanks of the fibres to spread loads over the jacketed flanks and reduce risk of damage to the fibres. The opposite sides of the spacer are further serrated to increase the resistance to fibre withdrawal provided by the gripping spacer, e.g. by biting onto the jacketed flanks of the fibres. Overall, the gripping spacer firmly and safely retains the fibres within the housing while reducing the risk of structural damage to either the polymer jacket or the optical waveguide core.

The housing 2 of Figures 1 and 2 can be formed of a suitable plastic material such as polyether ether ketone (PEEK) or polyvinyl chloride (PVC). Advantageously, these materials exhibit low compressibility and can protect the fibres from damage due to external loads. Similarly, the gripping element 6 can be formed of a suitable plastic material such as a polycarbonate, polyether ether ketone (PEEK), or polyvinyl chloride (PVC). Advantageously, these materials are generally harder than the polymer jackets of the optical fibres, promoting good fibre retention inside the housing. The gripping spacer and the housing may be manufactured by any one of or any combination of: 3D printing, machining, and moulding.

According to another option, the gripping spacer 6 could be made of a rubber or elastomeric material that is pulled into the matching channel 8. The pulling stretches the material and causes it contract transversely so that, when released to relax to its unstretched state, it expands transversely to grip the jacket and retain it by frictional interaction therewith.

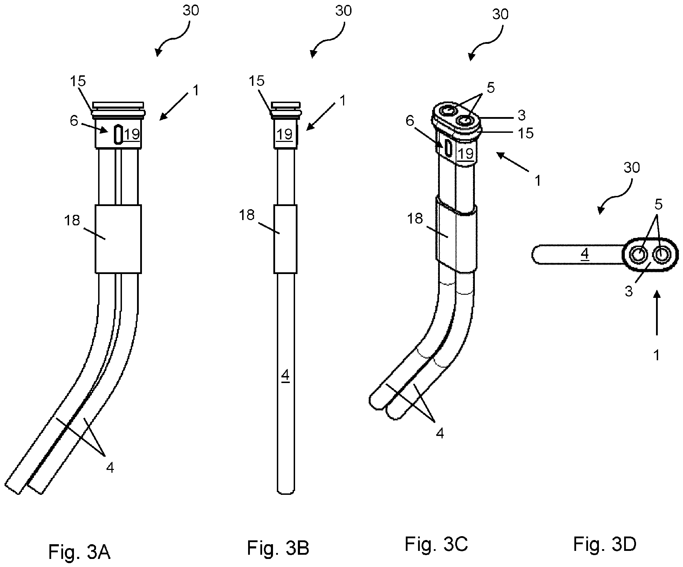

Figures 3A-3D show respectively a front elevation view, a side elevation view, a perspective view, and a plan view of a pair of terminated optical fibres 30. Identical features are referred to using the same reference numbers as in Figures 1 and 2. The terminated fibres of Figures 3A-3D additionally have a sleeve 18 located rearwards of the terminator 1 and wrapping around the optical fibres 4. The sleeve helps to maintain the fibre relative position introduced by the terminator 1 for a distance rearwards of the terminator and thus helps to prevent the fibres being accidently pulled apart in such a way that would damage them or the terminator, or cause the fibres end 5 to shift relative to the end face 3. Further rearwards of the sleeve, the fibres 4 can be more easily flexed, as shown in Figures 3A and 3C, thereby allowing the trailing ends of the fibres to be suitably positioned for coupling to a sensing unit, e.g. a reader card for sending light signals into and reading light signals from the optical fibres.

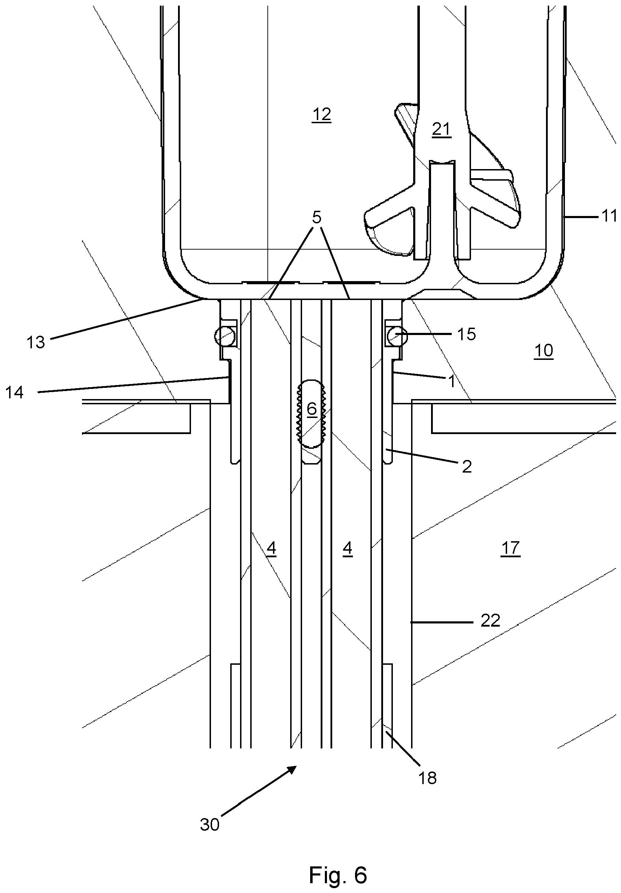

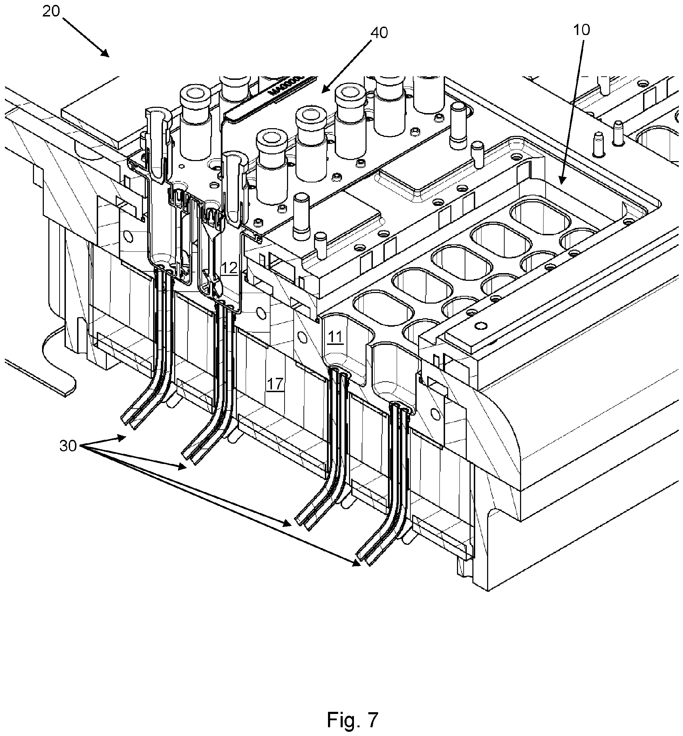

Figure 4 shows a partial plan view of the screening system 20 including a plurality of sample wells 11 for accommodating respective bioreaction vessels 12, Figure 5 shows a plan view of a multi-well screening station 10 of the screening system, Figure 6 shows a sectional view of a well 11 and a pair of the terminated optical fibres 30 of the screening system, and Figure 7 shows a perspective sectional view of part of the screening system.

Focusing initially on Figure 4, the screening system 20 can enable screening of large numbers of potential cell clones in parallel cell-culturing processes to identify the most suitable clones for larger-scale development processes. The sample wells in this example are arranged in multi-well screening stations 10 (four of which are shown in Figure 4), and the bioreaction vessels are similarly arranged in four, transparent plastic, bioreactor consumables 40 (only one of which is shown in Figure 4). The bioreactor consumables and the multi-well screening stations have matching (in this case 2x6) layouts such that each well 11 accommodates a respective bioreaction vessel 12. The screening system includes a temperature-controlled base 17 (not shown in Figure 4 but discussed below in relation to Figures 6 and 7) on which the wells are mounted. The base 17 in this example is formed of aluminium due to its high thermal conductivity. This can ensure that the temperature of the contents of the bioreactor consumables is reliably and accurately controlled.

Turning to Figure 5, each multi-well screening station 10 includes twelve sample wells 11 arranged in two rows of six wells. Each well is connected to a pair of the terminated fibres 30 of Figures 3A-3D such that the fibre end faces 5 are directed upwardly into the respective bioreaction vessel 12 which, in use, is located in the well, as shown in Figure 6. This can ensure a close contact between the fibre end faces and a base 13 of the respective bioreaction vessel, which can promote reliable interrogation of the contents of the bioreaction vessel by reducing a risk of compromised signal phase and amplitudes. This in turn helps to reduce a risk of loss of bioreaction control due to incorrect measurements. The screening system 20 of Figure 4 has in total sixteen screening stations 10 requiring 16x12=192 pairs of terminated fibres. However, the optical fibre terminator 1 allows these pairs to be terminated quickly, accurately and reliably, and in a way that is compatible with fibre replacement, should that be needed.

The sectional view of Figure 6 shows a single sample well 11 of one of the screening stations 10 mounted on the temperature-controlled base 17 and accommodating a single bioreaction vessel 12 of one of the bioreactor consumables 40. The vessel 12 has an agitator 21 for stirring and agitating its contents. The well has a base 13 and a docking port 14 formed in the base. The temperature-controlled base includes an aperture underneath each docking port leading to a channel 22 through the base. To mount a pair of the terminated optical fibres 30 to the well, trailing ends of the fibres are threaded through the docking port and then through the channel in the base. The terminator 1 is then docked in the docking port to secure the fibre end faces 5 such that they can reliably interrogate the contents of the bioreaction vessel. The O-ring 15 seals the terminator to the docking port to prevent accidental spills from the bioreaction vessel, condensation or cleaning liquids penetrating through the docking port. To improve the seal of the terminator to the docking port 14, the docking port may have a tapered profile that progressively engages the O-ring when the terminator is inserted therein.

As shown in Figure 7, the trailing ends of the terminated optical fibres 30 extend completely through the channels 22 formed in the temperature-controlled base 17 to project from the underside of the base. At this position, the trailing ends can be coupled a reader card to facilitate sending light signals into and reading light signals from the optical fibres. For example, a commercially available reader card such as the Presens^{TM} pH and DO reader card can be used. The reader card can be indexed from fibre to fibre to send to and read from each bioreaction vessel, whereby a single card can interrogate all the bioreaction vessels 12.

To replace individual malfunctioning or damaged fibres, first the respective bioreactor consumable 40 is removed from the screening station 10 to expose the pair of terminated fibres 30. Then, the fibres' trailing ends are pushed from underneath the screening system to cause the terminator 1 to protrude out of the well 11. This allows the terminator to be grasped and the pair of terminated fibres pulled out of the well. Once access to the terminator has been provided, the gripping spacer 6 can be removed and a selected fibre 4 can be withdrawn from the housing and replaced so that an end face of the replacement fibre is positioned at its predetermined spaced position relative to the other fibre and the housing end face, and the gripping spacer can be re-inserted to retain the fibres. Finally, the new pair of terminated fibres 30 can be connected to the screening station 20 in same manner as described in relation to Figure 6.

Advantageously, the terminator 1 of Figures 1 to 7 is easy to assemble, and requires no gluing or bonding to connect to the optical fibres, thereby facilitating replacement of individual damaged or malfunctioning fibres. Moreover, the terminator does not require the polymer jackets of the fibres to be pierced in order to grip the fibres, and therefore reduces the risk of structural damage to the optical waveguide cores. Rather, the terminator can firmly and safely retain the fibres via the gripping spacer. Thus, the terminator reduces costs and increases reliability compared to conventional fibre attachment solutions discussed above. Additionally, the terminator can be made compact and is thus useable even with small-scale and closely spaced bioreaction vessels.

The features disclosed in the description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

## Claims

1. An optical fibre terminator (1) for terminating two parallel optical fibres, each optical fibre comprising an optical waveguide core surrounded by a protective polymer jacket, the terminator including:
a housing (2) having a front face (3) and a backshell (19), and being configured for insertion thereinto of the two parallel optical fibres (4) so that respective end faces (5) of the two optical fibres are located at predetermined spaced positions relative to the front face and the two optical fibres extend rearwardly away from the front face through the backshell of the housing; and
a gripping spacer (6) insertable into a matching channel formed in the backshell behind the front face and between the predetermined spaced positions such that, on insertion, the gripping spacer grips jacketed flanks of the two optical fibres on opposite sides of the spacer to thereby space the fibres apart and retain the fibres in the housing with the fibre end faces locked at the predetermined spaced positions.

2. The optical fibre terminator (1) according to claim 1, wherein the gripping spacer (6) has serrated opposite sides (7) to grip the jacketed flanks of the inserted fibres (4).

3. The optical fibre terminator (1) according to claim 1 or 2, wherein the gripping spacer (6) is removably insertable into the housing (2).

4. The optical fibre terminator (1) according to any one of the previous claims, wherein the gripping spacer (6) has an obround cross section perpendicular to its insertion direction, the sides of the spacer which grip the jacketed flanks of the two optical fibres being the straight edges of the obround cross section.

5. The optical fibre terminator (1) according to any one of the previous claims, wherein the front face (3) of the housing (2) includes respective openings to receive the fibre end faces (5) and to define the predetermined spaced positions.

6. The optical fibre terminator (1) according to any one of the previous claims, wherein when the fibre end faces (5) are located at the predetermined spaced positions, the fibre end faces are flush with the front face (3) of the housing (2).

7. The optical fibre terminator (1) according to any one of the previous claims which is a duplex fibre terminator for terminating just two parallel optical fibres.

8. The optical fibre terminator (1) according to any one of the previous claims further including a sealing element (15) configured to seal the terminator to a docking port (14), wherein the sealing element is located in a matching groove (16) formed in the backshell (19) of the terminator (1).

9. A pair of terminated optical fibres (30) terminated with the terminator (1) according to any one of the previous claims.

10. The pair of terminated optical fibres (30) according to claim 9, wherein the polymer jackets are formed of polyethylene.

11. The pair of terminated optical fibres (30) according to claim 9 or 10, wherein each of the terminated optical fibres has a fibre diameter of 3 mm or less, and preferably of 2 mm or less.

12. The pair of terminated optical fibres (30) according to any one of claims 9 to 11, wherein a ratio of the centre-to-centre spacing between the end faces of the fibres at the predetermined spaced positions to the diameter of the fibres is 2 or less, and preferably 1.7 or less.

13. A combination of a screening system (20) and plural pairs of the terminated optical fibres (30) according to any one of claims 9 to 12, wherein:
the screening system includes a plurality of sample wells (11) for accommodating respective bioreaction vessels; and
each well includes a docking port (14) and has the terminator of a respective pair of the terminated optical fibres (30) docked therein such that the optical fibres of the pair can interrogate contents of a bioreaction vessel accommodated in the well via their fibre end faces.

14. The combination according to claim 13 as dependent on claim 8, wherein:
each terminator is sealed to its respective docking port via the respective sealing element (15).

15. The combination according to any one of claims 13 or 14, wherein the screening system (20) further includes a temperature-controlled base (17) on which the sample wells are supported, the optical fibres threading through apertures formed in the temperature-controlled base as they extend rearwardly away from the front faces of their terminators.

16. A method of assembling the combination according to any one of claims 13 to 15, the method including the steps of:
providing plural pairs of the terminated optical fibres (4); and
threading trailing ends of each pair of the terminated optical fibres (30) through the docking port (14) of the respective sample well (11) before inserting the respective terminator into the docking port such that the optical fibres can interrogate contents of a bioreaction vessel accommodated in the well via their fibre end faces.

17. The method according to claim 16 as dependent on claim 15, the method further including a step of threading the trailing ends of the terminated optical fibres (30) through the apertures formed in the temperature-controlled base after the trailing ends have been threaded through the docking ports.
